# EUROPEAN PATENT SPECIFICATION

(11) **EP 0 474 517 B1**
(45) Date of publication and mention of the grant of the patent: **18.11.1998**
(21) Application number: 91308205.3
(22) Date of filing: 09.09.1991
(51) Int. Cl.: A61K 31/59

(54) **Novel use of 1alpha-hydroxylated-19-nor-vitamin D compounds to treat psoriasis**
Neue Verwendung von 1-Alpha-hydroxylierten-19-nor-vitamin-D-Verbindungen zur Behandlung von Psoriasis
Nouvelle utilisation de composés 1-alpha-hydroxylés de la 19-nor vitamine D pour traiter le psoriasis

(30) Priority: 07.09.1990 US 579935
(43) Date of publication of application: 11.03.1992
(73) Proprietor: WISCONSIN ALUMNI RESEARCH FOUNDATION, Madison, WI 53705 (US)
(72) Inventor: DeLuca, Hector Floyd, Deerfield, Wisconsin 53531 (US)
(74) Representative: Ellis-Jones, Patrick George Armine

(56) References cited:
- EP-A- 0 387 077
- WO-A-89/10351
- TETRAHEDRON LETTERS, vol. 31, no. 13, 3rd April 1990, pages 1823-1824, Pergamon Press plc; K.L. PERLMAN et al.: "1alpha,25-dihydroxy-19-nor-vitamin D3, a novel vitamin D-related compound with potential therapeutic activity"

## Description

### Background of the Invention

The present invention relates to vitamin D compounds, and more particularly to the use of 1α-hydroxylated-19-nor-vitamin D compounds to treat psoriasis.

The D vitamins are very important agents for the control of calcium and phosphate metabolism in animals and humans, and have long been used as dietary supplements and in clinical practice to assure proper bone growth and development. It is now known that the in vivo activity of these vitamins, specifically of vitamin D₂ and D₃, is dependent on metabolism to hydroxylated forms. Thus, vitamin D₃ undergoes two successive hydroxylation reactions in vivo, leading first to 25-hydroxyvitamin D₃ and then to 1,25-dihydroxyvitamin D₃ and the latter is indeed thought to be the compound responsible for the well-known beneficial effects of vitamin D₃. Likewise, vitamin D₂, which is commonly used as a dietary supplement, undergoes an analogous hydroxylation sequence to its active forms, being first converted to 25-hydroxyvitamin D₂ (25-OH-D₂) and then to 1,25-dihydroxyvitamin D₂ (1,25-(OH)₂D₂). These facts are well established and well known in the art (see, for example, Suda et al. Biochemistry 8, 3515 (1969) and Jones et al. Biochemistry 14, 1250 (1975)).

Hollick, U. S. Patent No. 4,728,643 discloses a method of treating psoriasis with vitamin D compounds which in vitro cause cell differentiation. However 1α-hydroxylated vitamin D compounds, i.e. those compounds having only a hydroxyl group at the carbon 1 position and initially lacking a hydroxyl group at the carbon 24 or 25 positions, are relatively inactive in causing cell differentiation in vitro. Additionally, it is also well known that 1α-hydroxylated compounds are rapidly converted in vivo to 1α,25-dihydroxy compounds, e.g. 1α-hydroxyvitamin D₃ to 1α,25-dihydroxy-vitamin D₃, or if the 25 carbon position is blocked to 1α,24-dihydroxy compounds. Hollick et al, Science, Vol. 190, pages 576-578 (1975) and Hollick et al, J. of Clinical Endocrinology & Metabolism, Vol. 44., pages 595-598 (1977). For example, in PCT patent application number PCT/DK89/00079 filed April 7, 1989 and published November 2, 1989 under number WO89/10351 there is disclosed numerous side chain homologated vitamin D compounds lacking the hydroxyl group at the carbon 25 position in the side chain. It is disclosed therein that such compounds are converted in vivo to active compounds having a hydroxyl group at the carbon 25 position by enzymatic hydroxylation, and may thus be used for the treatment of psoriasis. Thus, the human body can rapidly convert relatively inactive 1α-hydroxylated vitamin D compounds to metabolites highly active in causing cell differentiation. There has, however, been a failure in the art to recognize the ability of 1α-hydroxylated-19-nor-vitamin D compounds to treat malignancies such as psoriasis.

Tetrahedron Letters, Vol 31, No. 13, pp 1823-4, 1990 discloses 1α,25-dihydroxy-19-nor-vitamin D₃ and its use in the treatment of malignancies while EP-A-0387077, which forms part of the state of the art by virtue of Article 54(3) EPC, discloses a class of 19-nor vitamin D compounds but none of these contains a triple bond in the side chain.

### Summary of the Invention

Compositions containing one or more 1α-hydroxylated-19-nor-vitamin D compounds with a triple bond in the side chain which compounds when administered to humans are converted to a metabolite, which metabolite in vitro has cell differentiation activity, together with a suitable carrier useful in the treatment of psoriasis are described. The treatment may be topical, oral or parenteral. Methods of employing the compositions are also disclosed. The compounds are present in the composition in an amount from about 0.01 µg/gm to about 100 µg/gm of the composition, and may be administered orally or parenterally in dosages of from about 0.01 µg/day to about 100 µg/day.

The compounds disclosed herein unexpectedly provide highly effective treatments for psoriasis without producing unwanted systemic or local side effects.

### Detailed Description of the Invention

The vitamin D compounds useful in the compositions of the present invention and for the treatment of psoriasis are those which are solely 1α-hydroxylated, i.e. those that do not initially have a hydroxyl group at the 24 or 25 carbon position in the side chain. Such 1α-hydroxylated compounds are readily converted to 1α,25-dihydroxy or 1α,24-dihydroxy compounds in vivo. These dihydroxy compounds are highly potent in inducing cellular differentiation, and the preferred compounds are those which induce cellular differentiation with minimal or no effect on either intestinal calcium absorption or bone calcium mobilization. Accordingly, specific preferred examples of vitamin D compounds defined by the above functions are those selected from the group consisting of 1α-hydroxy-19-nor-vitamin D compounds.

The 1α-19-nor-vitamin D compounds referred to herein are a class of 1α-hydroxylated vitamin D compounds in which the ring A exocyclic methylene group (carbon 19) typical of all vitamin D systems has been removed and replaced by two hydrogen atoms. Structurally these novel analogs are characterized by the general formula II shown below: 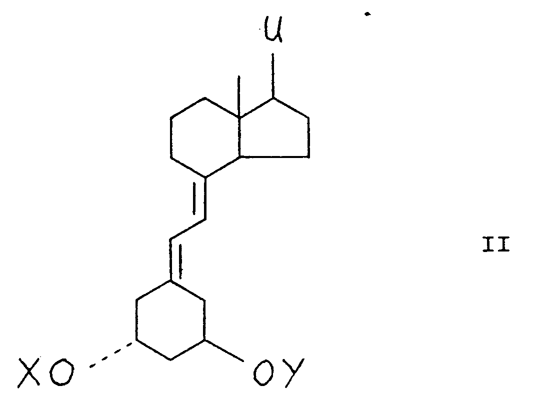 where X and Y are each selected from the group consisting of hydrogen, acyl, alkylsilyl and alkoxyalkyl, and where the group U represents the following side chain: 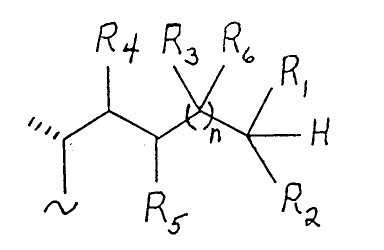 wherein R₁ and R₂ are each selected from the group consisting of alkyl, hydroxyalkyl and fluoroalkyl, deuteroalkyl or, when taken together represent the group -- (CH₂)ₘ -- where m is an integer having a value of from 2 to 5, R₃ is selected from the group consisting of hydrogen, deuterium, hydroxy, fluorine, O-acyl, alkyl, hydroxyalkyl and fluoroalkyl, R₆ is selected from the group consisting of hydrogen, deuterium, fluorine, alkyl, hydroxyalkyl and fluoroalkyl, or, R₃ and R₆ taken together represent double-bonded oxygen or double-bonded carbon, R₄ and R₅ taken together form a carbon-carbon triple bond i.e. a carbon-carbon triple bond is formed between the two carbon atoms, and wherein n is an integer having a value of from 1 to 5, and wherein the carbon at any one of positions 20, 22 or 23 in the side chain may be replaced by an O, S, or N atom with the proviso that when R₃ is other than hydroxy or O-acyl at least one of R₃ and R₆ is hydrogen or deuterium.

As used herein "alkyl" represents a straight-chain or branched hydrocarbon radical of 1 to 10 carbons in all its isomeric forms such as methyl, ethyl, propyl, isopropyl, butyl, isobutyl and pentyl, and the terms "hydroxyalkyl", "fluoroalkyl" and "deuteroalkyl" refer to such an alkyl radical substituted by one or more hydroxy or fluoro or deuterium groups respectively. An acyl group is an alkanoyl group of 1 to 6 carbons in all its isomeric forms, or an aroyl group, such as benzoyl, or halo-, nitro- or alkyl-substituted benzoyl groups, or a dicarboxylic acyl group such as oxalyl, malonyl, succinoyl, glutaroyl, or adipoyl. The term "aryl" signifies a phenyl-, or an alkyl-, nitro- or halo-substituted phenyl group.

It should be noted in this description that the term "24-dihomo" refers to the addition of two methylene groups at the carbon 24 position in the side chain. Likewise, the term "trihomo" refers to the addition of three methylene groups. Also, the term "26,27-dimethyl" refers to the addition of a methyl group at the carbon 26 and 27 positions so that for example R₁ and R₂ are ethyl groups. Likewise, the term "26,27-diethyl" refers to the addition of an ethyl group at the 26 and 27 positions so that R₁ and R₂ are propyl groups.

The preparation of 1α-hydroxy-19-nor-vitamin D compounds having the basic structure shown above in formula II can be accomplished by a common general method, using known vitamin D compounds as starting materials. Suitable starting materials are, for example, the vitamin D compounds of the general structure IV: 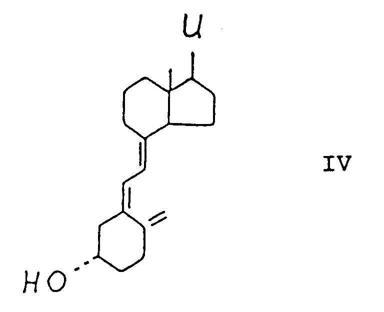 where U is any of the side chains as defined above. These vitamin D starting materials are known compounds, or compounds that can be prepared by known methods.

Using the procedure of DeLuca et al U.S. Patent 4,195,027, the starting material is converted to the corresponding 1α-hydroxy-3,5-cyclovitamin D derivative, having the general structure V below, where X³ represents hydrogen and Q represents an alkyl, preferably methyl: 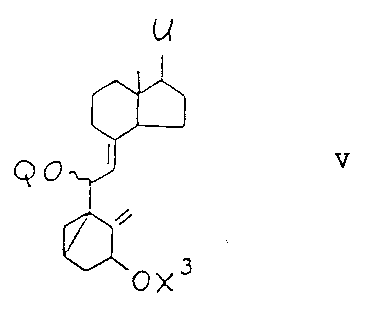 So as to preclude undesired reaction of the 1α-hydroxy group in subsequent steps, the hydroxy group is converted to the corresponding acyl derivative, i.e. the compound V shown above, where X³ represents an acyl group, using standard acylation procedures, such as treatment with an acyl anhydride or acyl halide in pyridine at room temperature or slightly elevated temperature (30-70°C). It should be understood also that whereas the process of this invention is illustrated here with acyl protection of hydroxy functions, alternative standard hydroxy-protecting groups can also be used, such as, for example, alkylsilyl or alkoxyalkyl groups. Such protecting groups are well-known in the art (e.g. trimethylsilyl, triethylsilyl, t.-butyldimethylsilyl, or tetrahydrofuranyl, methoxymethyl), and their use is considered a routine modification of experimental detail within the scope of the process of this invention.

The derivative as obtained above is then reacted with osmium tetroxide, to produce the 10,19-dihydroxy analog, VI (where X³ is acyl), which is subjected to diol cleavage using sodium metaperiodate or similar vicinal diol cleavage reagents (e.g. lead tetraacetate) to obtain the 10-oxo-intermediate, having the structure VII below (where X³ is acyl): 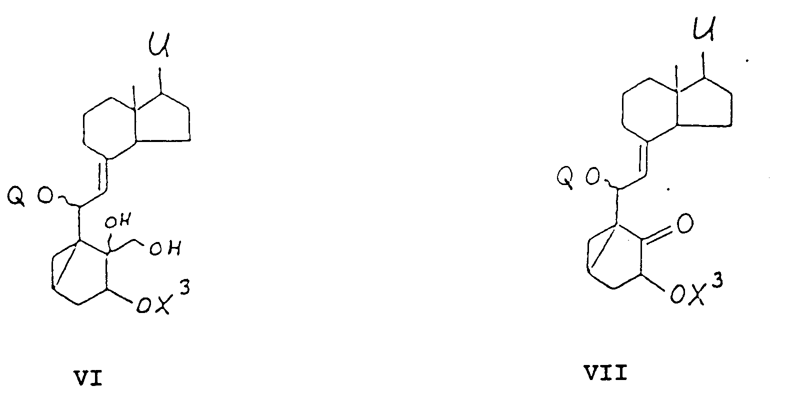 These two consecutive steps can be carried out according to the procedures given by Paaren et al. (J. Org. Chem. 48, 3819 (1983)). If the side chain unit, U carries vicinal diols (e.g. 24,25-dihydroxy- or 25,26-dihydroxy, etc.), these, of course, also need to be protected, e.g. via acylation, silylation, or as the isopropylidene derivative prior to the periodate cleavage reactions.

In most cases, the acylation of the 1α-hydroxy group as mentioned above will simultaneously effect the acylation of side chain hydroxy functions, and these acylation conditions can, of course, be appropriately adjusted (e.g. elevated temperatures, longer reaction times) so as to assure complete protection of side chain vicinal diol groupings.

The next step of the process comprises the reduction of the 10-oxo-group to the corresponding 10-alcohol having the structure VIII shown below (where X³ is acyl and Y³ represents hydroxy). When X³ is acyl, this reduction is carried out conveniently in an organic solvent at from about 0°C to about room temperature, using NaBH₄ or equivalent hydride reducing agents, selective for the reduction of carbonyl groups without cleaving ester functions. Obviously, when X³ is a hydroxy-protecting group that is stable to reducing agents, any of the other hydride reducing agents (e.g. LiAlH₄, or analogous reagents) may be employed also.

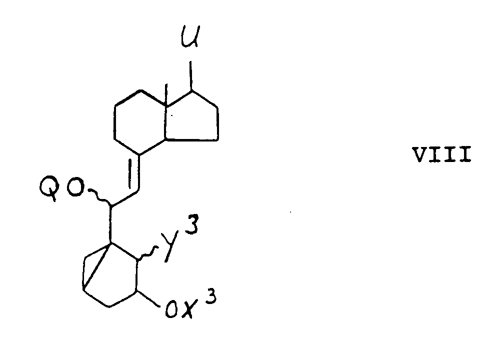

The 10-hydroxy intermediate is then treated with an alkyl-or arylsulfonylhalide (e.g. methanesulfonylchloride) in a suitable solvent (e.g. pyridine) to obtain the corresponding 10-0-alkyl-or arylsulfonyl derivative (the compound having the structure shown VIII above, where Y³ is alkyl-SO₂O-, or aryl-SO₂O-, and this sulfonate intermediate is then directly reduced, with lithium aluminum hydride, or the analogous known lithium aluminum alkyl hydride reagents in an ether solvent, at a temperature ranging from O°C to the boiling temperature of the solvent, thereby displacing the sulfonate group and obtaining the 10-deoxy derivative, represented by the structure VIII above, where X³ and Y³ are both hydrogen. As shown by the above structure, a 1-0-acyl function in the precursor compound VII is also cleaved in this reduction step to produce the free 1α-hydroxy function, and any 0-acyl protecting group in the side chain would, of course, likewise be reduced to the corresponding free alcohol function, as is well understood in the art. If desired, the hydroxy groups at C-1 (or hydroxy groups in the side chain) can be reprotected by acylation or silylation or ether formation to the corresponding acyl, alkylsilyl or alkoxyalkyl derivative, but such protection is not required. Alternative hydroxy-protecting groups, such as alkylsilyl or alkoxyalkyl groups would be retained in this reduction step, but can be removed, as desired, at this or later stages in the process by standard methods known in the art.

The above 1α-hydroxy-10-deoxy cyclovitamin D intermediate is next solvolyzed in the presence of a low-molecular weight organic acid, using the conditions of DeLuca et al U.S. Patents 4,195,027 and 4,260,549. When the solvolysis is carried out in acetic acid, for example, there is obtained a mixture of 1α-hydroxy-19-nor-vitamin D 3-acetate and 1α-hydroxy-19-nor-vitamin D 1-acetate (compounds IX and X, below), and the analogous 1- and 3-acylates are produced, when alternative acids are used for solvolysis. 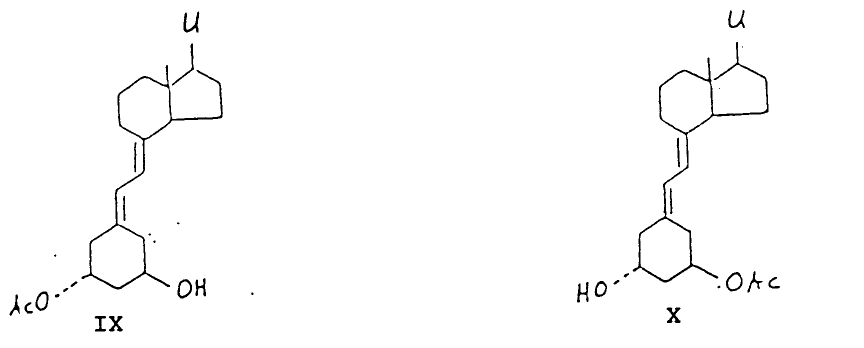 Direct basic hydrolysis of this mixture under standard conditions then produces the desired 1α-hydroxy-19-nor-vitamin D compounds of structure II above (where X¹ and Y¹ are both hydrogen). Alternatively, the above mixture of monoacetates may also be separated (e.g. by high pressure liquid chromatography) and the resulting 1-acetate and 3-acetate isomers may be subjected separately to hydroxysis to obtain the same final product from each, namely the 1α-hydroxy-19-nor-vitamin D compounds of structure II. Also the separated monoacetates of structure IX or X or the free 1,3-dihydroxy compound can, of course, be reacylated according to standard procedures with any desired acyl group, so as to produce the product of structure II above, where X¹ and Y¹ represent acyl groups which may be the same or different.

Compositions for use in the above-mentioned treatment of psoriasis comprise an effective amount of one or more 1α-hydroxy-19-nor-vitamin D compounds as defined by the above formula II as the active ingredient, and a suitable carrier. An effective amount of such compounds for use in accordance with this invention is typically from 0.01 µg to 100 µg per gm of composition, and may be administered topically, orally or parenterally in dosages of from, say, 0.1 µg/day to 100 µg/day.

The compounds may be formulated as creams, lotions, ointments, topical patches, pills, capsules or tablets, or in liquid form as solutions, emulsions, dispersions or suspensions in pharmaceutically innocuous and acceptable solvent or oils, and such preparations may contain in addition other pharmaceutically innocuous or beneficial components, such as antioxidants or preserving agents, stabilising, wetting or emulsifying agents, solution promoters, coloring agents, binders or coating materials.

The compositions of this invention are typically formulated as a foam (which may contain a propellant), a stick, a cleansing pad, an impregnated wipe, a face pack, a shaving foam or an after shave, but preferably as creams, lotions or ointments by choice of appropriate carriers. Suitable carriers may be solid or liquid and include vegetable or mineral oils such as corn starch, lactose, sucrose, peanut oil, olive oil and sesame oil, propylene glycol, white petrolatum (white soft paraffin), branched chain fats or oils, animal fats and high molecular weight alcohols (greater than C₁₂). The preferred carriers are those in which the active ingredient is soluble. Thickening agents (so that the composition is in the form of an ointment, cream, lotion or gel), other active cosmetic ingredients including anti-wrinkle agents and anti-grease agents along with additives such as surfactants, soaps, bath additives, organic solvents, emulsifiers, stabilizers and antioxidants may also be included as well as agents imparting color or fragrance if desired.

Creams are preferably formulated from a mixture of mineral oil, self-emulsifying beeswax and water in which mixture the active ingredient, dissolved in a small amount of an oil such as almond oil, is admixed. A typical example of such a cream is one which includes about 40 parts water, about 20 parts beeswax, about 40 parts mineral oil and about 1 part almond oil.

Ointments may be formulated by mixing a solution of the active ingredient in a vegetable oil such as almond oil with warm soft paraffin and allowing the mixture to cool. A typical example of such an ointment is one which includes about 30% almond oil and about 70% white soft paraffin by weight.

Lotions may be conveniently prepared by dissolving the active ingredient, in a suitable high molecular weight alcohol such as propylene glycol or polyethylene glycol.

The compounds may be administered topically, as oral doses, or parenterally by injection or infusion of suitable sterile solutions. The compounds are advantageously administered in amounts sufficient to effect the differentiation of promyelocytes to normal macrophages. Dosages as described above are suitable, it being understood that the amounts given are to be adjusted in accordance with the severity of the disease, and the condition and response of the subject as is well understood in the art. If a solid carrier is used the dosage form of the compounds is typically tablets, capsules, powders, troches or lozenges. If a liquid carrier is used, soft gelatin capsules, or syrup or liquid suspensions, emulsions or solutions may be the dosage form.

### Biological activity of 1α-Hydroxy-19-Nor-Vitamin D Compounds

The 19-nor compounds exhibit a pattern of biological activity having high potency in promoting the differentiation of malignant cells and little or no activity in calcifying bone tissue.

In this connection reference is made to the tests on differentiation of HL-60 cells and calcification activity for 1α,25-dihydroxy vitamin D₃ and its 19-nor analogu given in EP-A-387077.

It should be specifically noted that 1α-hydroxy-19-nor-vitamin D₃ is expected to be less active than 1α,25-dihydroxy-19-nor-vitamin D₃ in causing differentiation of HL60 cells in vitro. However, in vivo it is well established that 1α-hydroxy-vitamin D₃ is rapidly converted to 1α,25-dihydroxy-vitamin D₃, Hollick et al, Science, Vol. 190, pages 576-578 (1975) and Holick et al, Journal of Clinical Endocrinology & Metabolism, Vol. 44, pages 595-598 (1977). Thus, it is clear that the human body can rapidly convert the relatively inactive 1α-hydroxylated-19-nor-vitamin D compounds to metabolites highly active in causing cell differentiation. This in vivo capability makes possible the treatment of malignancies such as psoriasis with 1α-hydroxylated-19-nor-vitamin D compounds that do not initially have a hydroxyl group at the 24 or 25 carbon position in the side chain.

## Claims (Claims for the following Contracting State(s): AT, BE, CH, DE, DK, FR, GB, IT, LI, LU, NL, SE)

1. Use of a compound of formula I for the manufacture of a medicament for the treatment of psoriasis wherein formula I is: 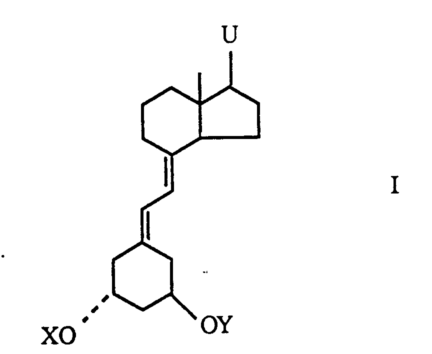 where X and Y are each independently hydrogen, acyl alkylsilyl or alkoxyalkyl, and where U is selected from a side chain of the formula 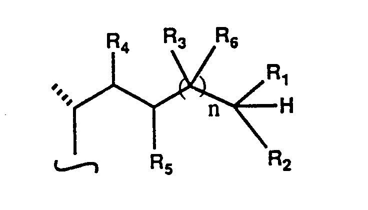 wherein R₁ and R₂ are each independently alkyl, deuteroalkyl, hydroxyalkyl or fluoroalkyl, or, when taken together represent the group --(CH₂)ₘ -- where m is an integer having a value of from 2 to 5, R₃ is hydrogen, deuterium, hydroxy, fluorine, O-acyl, alkyl, hydroxyalkyl or fluoroalkyl, R₆ is hydrogen, deuterium, fluorine, alkyl, hydroxyalkyl or fluoroalkyl, or, R₃ and R₆ taken together represent double-bonded oxygen or double-bonded carbon, R₄ and R₅ taken together form a carbon-carbon triple bond, and wherein n is an integer having a value of from 1 to 5 and wherein the carbon at any one of positions 20, 22, or 23 in the side chain may be replaced by an O, S, or N atom, with the proviso that when R₃ is other than hydroxy or O-acyl at least one of R₃ and R₆ is hydrogen or deuterium.

2. Use according to claim 1 wherein R₃ is hydroxy or O-acyl.

3. Use according to claim 1 or 2 wherein the medicament contains 0.01 µg to 100 µg of the compound per gram of the medicament.

4. Use according to any one of claims 1 to 3 wherein the medicament is administrable to a patient by oral or parenteral means, or by topical means when R₃ is hydroxy.

5. Use according to any one of the preceding claims wherein the medicament is used to provide an amount of the said compound of 0.1 µg/day to 100 µg/day.

6. A composition suitable for oral or parenteral treatment of psoriasis which comprises a compound of formula I as defined in claim 1 or 2 and an appropriate carrier.

7. A composition suitable for topical treatment of psoriasis which comprises a compound of formula I as defined in claim 1 where R₃ is hydroxy and an appropriate carrier.

8. A composition according to claim 6 or 7 which contains 0.01 µg to 100 µg of said compound per gram of the composition.

9. Use of a 1α-hydroxylated-19-nor-vitamin D compound of formula I as defined in claim 1 or 2 which compound upon administration to humans is converted to a metabolite and said metabolite in vitro will cause differentiation in a cell line for the manufacture of a medicament for the treatment of psoriasis.

10. Use according to claim 9 wherein said cell line is a U937 cell line, a HL60 cell line or a M1 cell line.

## Claims (Claims for the following Contracting State(s): ES)

1. A process for the manufacture of a medicament for the treatment of psoriasis which comprises mixing a compound of formula I 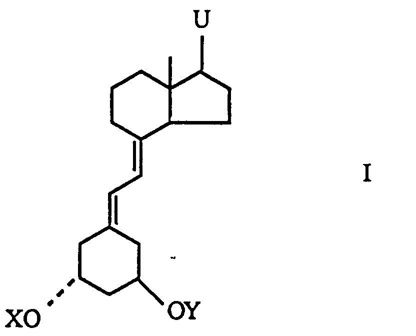 where X and Y are each independently hydrogen, acyl alkylsilyl or alkoxyalkyl, and where U is selected from a side chain of the formula. 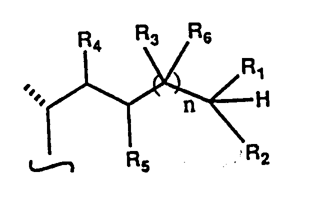 wherein R₁ and R₂ are each independently alkyl, deuteroalkyl, hydroxyalkyl or fluoroalkyl, or, when taken together represent the group -- (CH₂)ₘ -- where m is an integer having a value of from 2 to 5, R₃ is hydrogen, deuterium, hydroxy, fluorine, O-acyl, alkyl, hydroxyalkyl or fluoroalkyl, R₆ is hydrogen, deuterium, fluorine, alkyl, hydroxyalkyl or fluoroalkyl, or, R₃ and R₆ taken together represent double-bonded oxygen or double-bonded carbon, R₄ and R₅ taken together form a carbon-carbon triple bond, and wherein n is an integer having a value of from 1 to 5 and wherein the carbon at any one of positions 20, 22, or 23 in the side chain may be replaced by an O, S, or N atom, with the proviso that when R₃ is other than hydroxy or O-acyl at least one of R₃ or R₆ is hydrogen or deuterium with an appropriate carrier.

2. A process according to claim 1 wherein R₃ is hydroxy or O-acyl.

3. A process according to claim 1 or 2 wherein the medicament contains 0.01 µg to 100 µg of the compound per gram of the medicament.

4. A process according to any one of claims 1 to 3 wherein the medicament is administrable to a patient by oral or parenteral means, or by topical means when R₃ is hydroxy.

5. Process according to any one of the preceding claims wherein the medicament is used to provide an amount of the said compound of 0.1 µg/day to 100 µg/day.

6. A process for the manufacture of a medicament for the treatment of psoriasis which comprises mixing a 1α-hydroxylated-19-nor-vitamin D compound of formula I as defined in claim 1 or 2 which compound upon administration to humans is converted to a metabolite and said metabolite in vitro will cause differentiation in a cell line, with an appropriate carrier.

## Claims (Claims for the following Contracting State(s): GR)

1. Use of a compound of formula I for the manufacture of a medicament for the treatment of psoriasis wherein formula I is: 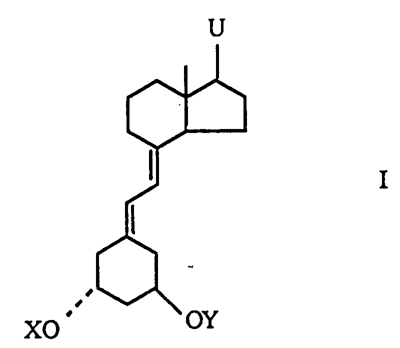 where X and Y are each independently hydrogen, acyl alkylsilyl or alkoxyalkyl, and where U is selected from a side chain of the formula 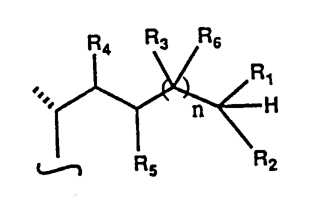 wherein R₁ and R₂ are each independently alkyl, deuteroalkyl, hydroxyalkyl or fluoroalkyl, or, when taken together represent the group -- (CH₂)ₘ -- where m is an integer having a value of from 2 to 5, R₃ is hydrogen, deuterium, hydroxy, fluorine, O-acyl, alkyl, hydroxyalkyl or fluoroalkyl, R₆ is hydrogen, deuterium, fluorine, alkyl, hydroxyalkyl or fluoroalkyl, or, R₃ and R₆ taken together represent double-bonded oxygen or double-bonded carbon, R₄ and R₅ taken together form a carbon-carbon triple bond, and wherein n is an integer having a value of from 1 to 5 and wherein the carbon at any one of positions 20, 22, or 23 in the side chain may be replaced by an O, S, or N atom, with the proviso that when R₃ is other than hydroxy or O-acyl at least one of R₃ and R₆ is hydrogen or deuterium.

2. Use according to claim 1 wherein R₃ is hydroxy or 0-acyl.

3. Use according to claim 1 or 2 wherein the medicament contains 0.01 µg to 100 µg of the compound per gram of the medicament.

4. Use according to any one of claims 1 to 3 wherein the medicament is administrable to a patient by oral or parenteral means, or by topical means when R₃ is hydroxy.

5. Use according to any one of the preceding claims wherein the medicament is used to provide an amount of the said compound of 0.1 µg/day to 100 µg/day.

6. Use of a 1α-hydroxylated-19-nor-vitamin D compound of formula I as defined in claim 1 or 2 which compound upon administration to humans is converted to a metabolite and said metabolite in vitro will cause differentiation in a cell line for the manufacture of a medicament for the treatment of psoriasis.

7. Use according to claim 6 wherein said cell line is a U937 cell line, a HL60 cell line or a M1 cell line.

8. A process for preparing a composition suitable for oral or parenteral treatment of psoriasis which comprises a compound of formula I as defined in claim 1 or 2 and an appropriate carrier which comprises mixing the compound of formula I with the carrier.

9. A process for preparing a composition suitable for topical treatment of psoriasis which comprises a compound of formula I as defined in claim 1 where R₃ is hydroxy and an appropriate carrier which comprises mixing the compound of formula I with the carrier.

10. A process according to claim 8 or 9 in which the composition contains 0.01 µg to 100 µg of said compound per gram of the composition.

11. A process for the manufacture of a medicament for the treatment of psoriasis which comprises mixing a compound of formula I as defined in claim 1 with an appropriate carrier.

## Patentansprüche (Patentansprüche für folgende(n) Vertragsstaat(en): AT, BE, CH, DE, DK, FR, GB, IT, LI, LU, NL, SE)

1. Verwendung einer Verbindung der Formel I zur Herstellung eines Medikaments zur Behandlung von Psoriasis 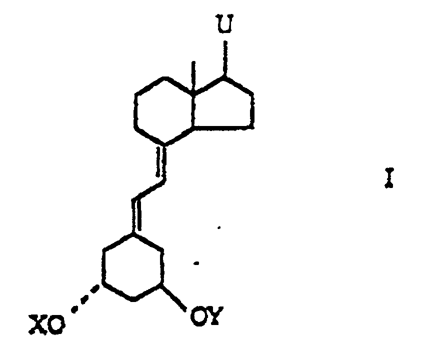 worin X und Y unabhängig voneinander Wasserstoff, Acyl, Alkylsilyl oder Alkoxyalkyl bedeuten, und U eine Seitenkette der Formel sind, 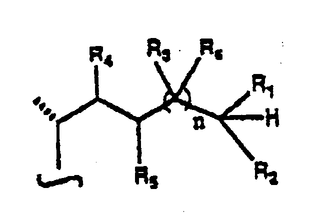 worin R₁ und R₂ unabhängig voneinander Alkyl, Deuteroalkyl, Hydroxyalkyl oder Fluoralkyl bedeuten, oder zusammen die Gruppe -(CH₂)ₘ- bedeuten, worin m eine ganze Zahl von 2 bis 5 ist, R₃ Wasserstoff, Deuterium, Hydroxy, Fluor, O-Acyl, Alkyl, Hydroxyalkyl oder Fluoralkyl ist, R₆ Wasserstoff, Deuterium, Fluor, Alkyl, Hydroxyalkyl oder Fluoralkyl ist, oder R₃ und R₆ zusammen doppeltgebundenen Sauerstoff oder doppeltgebundenen Kohlenstoff bedeuten, R₄ und R₅ zusammen eine Kohlenstoff-Kohlenstoff-Dreifachbindung bilden, und n eine ganze Zahl von 1 bis 5 ist, und worin das Kohlenstoffatom in einer der Stellungen 20, 22 oder 23 in der Seitenkette durch ein O-, S- oder N-Atom ersetzt sein kann, mit der Maßgabe, daß, wenn R₃ von Hydroxy oder O-Acyl verschieden ist, mindestens einer der Reste R₃ und R₆ Wasserstoff oder Deuterium ist.

2. Verwendung nach Anspruch 1, dadurch gekennzeichnet, daß R₃ Hydroxy oder O-Acyl ist.

3. Verwendung nach Anspruch 1 oder 2, dadurch gekennzeichnet, daß das Arzneimittel 0,01 µg bis 100 µg der Verbindung pro Gramm Arzneimittel enthält.

4. Verwendung nach einen der Ansprüche 1 bis 3, dadurch gekennzeichnet, daß das Arzneimittel an einen Patienten oral oder parenteral verabreichbar ist, oder, wenn R₃ Hydroxy ist, topisch.

5. Verwendung nach einen der vorhergehenden Ansprüche, dadurch gekennzeichnet, daß das Arzneimittel so verwendet wird, daß die Verbindung in einer Menge von 0,1 µg/Tag bis 100 µg/Tag bereitgestellt wird.

6. Zusammensetzung zur oralen oder parenteralen Behandlung von Psoriasis, dadurch gekennzeichnet, daß sie eine Verbindung der Formel I nach Anspruch 1 oder 2 und einen geeigneten Träger umfaßt.

7. Zusammensetzung zur topischen Behandlung von Psoriasis, dadurch gekennzeichnet, daß sie eine Verbindung der Formel I nach Anspruch 1, worin R₃ Hydroxy ist, und einen geeigneten Träger umfaßt.

8. Zusammensetzung nach Anspruch 6 oder 7, dadurch gekennzeichnet, daß sie 0,01 µg bis 100 µg der Verbindung pro Gramm der Zusammensetzung enthält.

9. Verwendung einer 1α-hydroxylierten 19-Norvitamin D-Verbindung der Formel I nach Anspruch 1 oder 2, dadurch gekennzeichnet, daß die Verbindung bei der Verabreichung an Menschen in einen Metaboliten überführt wird, und der Metabolit in vitro eine Differenzierung einer Zellinie zur Herstellung eines Arzneimittels zur Behandlung von Psoriasis verursacht.

10. Verwendung nach Anspruch 9, dadurch gekennzeichnet, daß die Zellinie eine U937-Zellinie, eine HL60-Zellinie oder eine M1-Zellinie ist.

## Patentansprüche (Patentansprüche für folgende(n) Vertragsstaat(en): ES)

1. Verfahren zur Herstellung eines Arzneimittels zur Behandlung von Psoriasis, dadurch gekennzeichnet, daß man eine Verbindung der Formel I 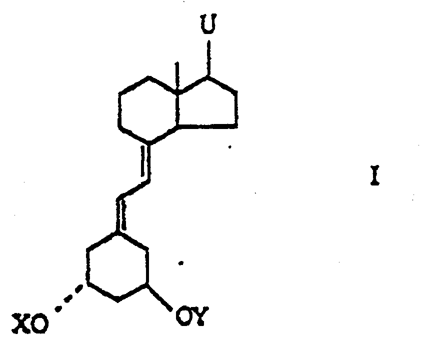 worin X und Y unabhängig voneinander Wasserstoff, Acyl, Alkylsilyl oder Alkoxyalkyl bedeuten, und U eine Seitenkette der Formel sind, 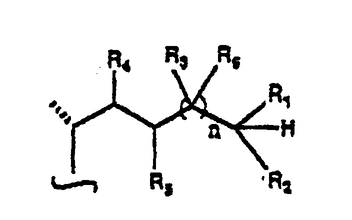 worin R₁ und R₂ unabhängig voneinander Alkyl, Deuteroalkyl, Hydroxyalkyl oder Fluoralkyl bedeuten, oder zusammen die Gruppe -(CH₂)ₘ- bedeuten, worin m eine ganze Zahl von 2 bis 5 ist, R₃ Wasserstoff, Deuterium, Hydroxy, Fluor, O-Acyl, Alkyl, Hydroxyalkyl oder Fluoralkyl ist, R₆ Wasserstoff, Deuterium, Fluor, Alkyl, Hydroxyalkyl oder Fluoralkyl ist, oder R₃ und R₆ zusammen doppeltgebundenen Sauerstoff oder doppeltgebundenen Kohlenstoff bedeuten, R₄ und R₅ zusammen eine Kohlenstoff-Kohlenstoff-Dreifachbindung bilden, und n eine ganze Zahl von 1 bis 5 ist, und worin das Kohlenstoffatom in einer der Stellungen 20, 22 oder 23 in der Seitenkette durch ein O-, S- oder N-Atom ersetzt sein kann, mit der Maßgabe, daß, wenn R₃ von Hydroxy oder O-Acyl verschieden ist, mindestens einer der Reste R₃ und R₆ Wasserstoff oder Deuterium ist, mit einem geeigneten Träger mischt.

2. Verfahren nach Anspruch 1, dadurch gekennzeichnet, daß R₃ Hydroxy oder O-Acyl ist.

3. Verfahren nach Anspruch 1 oder 2, dadurch gekennzeichnet, daß das Arzneimittel 0,01 µg bis 100 µg der Verbindung pro Gramm Arzneimittel enthält.

4. Verfahren nach einem der Ansprüche 1 bis 3, dadurch gekennzeichnet, daß das Arzneimittel an einen Patienten oral oder parenteral verabreichbar ist, oder, wenn R₃ Hydroxy ist, topisch.

5. Verfahren nach einem der vorhergehenden Ansprüche, dadurch gekennzeichnet, daß das Arzneimittel so verwendet wird, daß die Verbindung in einer Menge von 0,1 µg/Tag bis 100 µg/Tag bereitgestellt wird.

6. Verfahren zur Herstellung eines Arzneimittels zur Behandlung von Psoriasis, dadurch gekennzeichnet, daß man eine 1α-hydroxylierte 19-Norvitamin D-Verbindung der Formel I nach Anspruch 1 oder 2, die bei der Verabreichung an Menschen in einen Metaboliten übergeführt wird, und der Metabolit in vitro die Differenzierung einer Zellinie verursacht, mit einen geeigneten Träger mischt.

## Patentansprüche (Patentansprüche für folgende(n) Vertragsstaat(en): GR)

1. Verwendung einer Verbindung der Formel I zur Herstellung eines Medikaments zur Behandlung von Psoriasis 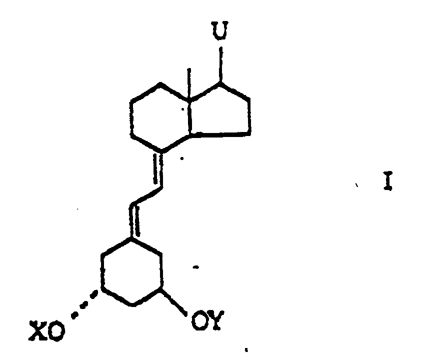 worin X und Y unabhängig voneinander Wasserstoff, Acyl, Alkylsilyl oder Alkoxyalkyl bedeuten, und U eine Seitenkette der Formel sind, 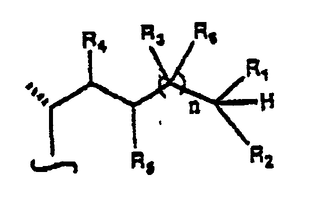 worin R₁ und R₂ unabhängig voneinander Alkyl, Deuteroalkyl, Hydroxyalkyl oder Fluoralkyl bedeuten, oder zusammen die Gruppe -(CH₂)ₘ- bedeuten, worin m eine ganze Zahl von 2 bis 5 ist, R₃ Wasserstoff, Deuterium, Hydroxy, Fluor, O-Acyl, Alkyl, Hydroxyalkyl oder Fluoralkyl ist, R₆ Wasserstoff, Deuterium, Fluor, Alkyl, Hydroxyalkyl oder Fluoralkyl ist, oder R₃ und R₆ zusammen doppeltgebundenen Sauerstoff oder doppeltgebundenen Kohlenstoff bedeuten, R₄ und R₅ zusammen eine Kohlenstoff-Kohlenstoff-Dreifachbindung bilden, und n eine ganze Zahl von 1 bis 5 ist, und worin das Kohlenstoffatom in einer der Stellungen 20, 22 oder 23 in der Seitenkette durch ein O-, S- oder N-Atom ersetzt sein kann, mit der Maßgabe, daß, wenn R₃ von Hydroxy oder O-Acyl verschieden ist, mindestens einer der Reste R₃ und R₆ Wasserstoff oder Deuterium ist.

2. Verwendung nach Anspruch 1, dadurch gekennzeichnet, daß R₃ Hydroxy oder O-Acyl ist.

3. Verwendung nach Anspruch 1 oder 2, dadurch gekennzeichnet, daß das Arzneimittel 0,01 µg bis 100 µg der Verbindung pro Gramm Arzneimittel enthält.

4. Verwendung nach einem der Ansprüche 1 bis 3, dadurch gekennzeichnet, daß das Arzneimittel an einen Patienten oral oder parenteral verabreichbar ist, oder, wenn R₃ Hydroxy ist, topisch.

5. Verwendung nach einem der vorhergehenden Ansprüche, dadurch gekennzeichnet, daß das Arzneimittel so verwendet wird, daß die Verbindung in einer Menge von 0,1 µg/Tag bis 100 µg/Tag bereitgestellt wird.

6. Verwendung einer 1α-hydroxylierten 19-Norvitamin D-Verbindung der Formel I nach Anspruch 1 oder 2, dadurch gekennzeichnet, daß die Verbindung bei der Verabreichung an Menschen in einen Metaboliten überführt wird, und der Metabolit in vitro eine Differenzierung einer Zellinie zur Herstellung eines Arzneimittels zur Behandlung von Psoriasis verursacht.

7. Verwendung nach Anspruch 6, dadurch gekennzeichnet, daß die Zellinie eine U937-Zellinie, eine HL60-Zellinie oder eine M1-Zellinie ist.

8. Verfahren zur Herstellung einer zur oralen oder parenteralen Behandlung von Psoriasis geeigneten Zusammensetzung, die eine Verbindung der Formel I nach Anspruch 1 oder 2 und einen geeigneten Träger umfaßt, dadurch gekennzeichnet, daß man die Verbindung der Formel I mit dem Träger mischt.

9. Verfahren zur Herstellung einer zur topischen Behandlung von Psoriasis geeigneten Zusammensetzung, die eine Verbindung der Formel I nach Anspruch 1, worin R₃ Hydroxy ist, und einen geeigneten Träger umfaßt, dadurch gekennzeichnet, daß man die Verbindung der Formel I mit dem Träger mischt.

10. Verfahren nach Anspruch 8 oder 9, dadurch gekennzeichnet, daß die Zusammensetzung 0,01 µg bis 100 µg der Verbindung pro Gramm der Zusammensetzung enthält.

11. Verfahren zur Herstellung eines Arzneimittels zur Behandlung von Psoriasis, dadurch gekennzeichnet, daß man eine Verbindung der Formel I nach Anspruch 1 mit einem geeigneten Träger mischt.

## Revendications (Revendications pour l'(les) Etat(s) contractant(s) suivant(s): AT, BE, CH, DE, DK, FR, GB, IT, LI, LU, NL, SE)

1. Utilisation d'un composé de formule I pour la fabrication d'un médicament destiné au traitement du psoriasis, la formule I étant la suivante : 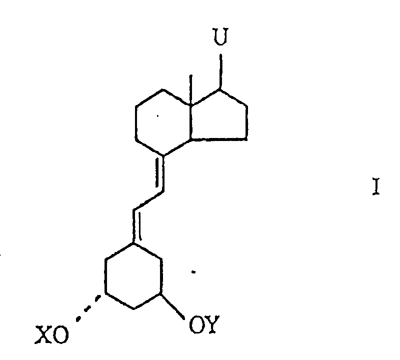 dans laquelle X et Y représentent chacun, indépendamment, un atome d'hydrogène ou un groupe acyle, alkylsilyle ou alcoxyalkyle, et U représente une chaîne latérale de formule 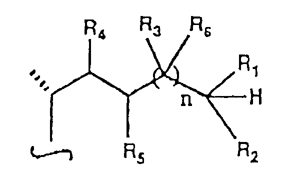 dans laquelle R₁ et R₂ représentent chacun, indépendamment, un groupe alkyle, deutéroalkyle, hydroxyalkyle ou fluoroalkyle, ou bien représentent conjointement un groupe ―(CH₂)ₘ― où m représente un nombre entier valant de 2 à 5, R₃ représente un atome d'hydrogène, de deutérium ou de fluor ou un groupe hydroxyle, O-acyle, alkyle, hydroxyalkyle ou fluoroalkyle, R₆ représente un atome d'hydrogène, de deutérium ou de fluor ou un groupe alkyle, hydroxyalkyle ou fluoroalkyle, ou bien R₃ et R₆ représentent conjointement un atome d'oxygène a double liaison ou un atome de carbone à double liaison, R₄ et R₅ représentent conjointement une triple liaison carbone-carbone et n représente un nombre entier valant de 1 à 5, et I'atome de carbone occupant n'importe laquelle des positions 20, 22 et 23 dans la chaîne latérale peut être remplacé par un atome d'oxygène, de soufre ou d'azote, sous réserve que, si R₃ représente autre chose qu'un groupe hydroxyle ou O-acyle, au moins l'un des R₃ et R₆ représente un atome d'hydrogène ou de deutérium.

2. Utilisation conforme à la revendication 1, pour laquelle R₃ représente un groupe hydroxyle ou O-acyle.

3. Utilisation conforme à la revendication 1 ou 2, où le médicament contient de 0,01 µg à 100 µg du composé par gramme de médicament.

4. Utilisation conforme à l'une des revendications 1 à 3, où le médicament peut être administré à un patient par voie orale ou parentérale, ou encore en usage externe si R₃ représente un groupe hydroxyle.

5. Utilisation conforme à l'une des revendications précédentes, où l'on emploie le médicament de façon à apporter ledit composé en une quantité de 0,1 µg/jour à 100 µg/jour.

6. Composition appropriée pour le traitement du psoriasis par voie orale ou parentérale, qui contient un composé de formule I, définie dans la revendication 1 ou 2, et un véhicule convenable.

7. Composition appropriée, en usage externe, pour le traitement du psoriasis, qui contient un composé de formule I, définie dans la revendication 1, où R₃ représente un groupe hydroxyle, et un véhicule convenable.

8. Composition conforme à la revendication 6 ou 7, qui contient de 0,01 µg à 100 µg dudit composé par gramme de composition.

9. Utilisation d'une 1α-hydroxy-19-nor-vitamine D, composé de formule I définie dans la revendication 1 ou 2, lequel composé est converti, après administration chez l'homme, en un métabolite qui provoque in vitro la différenciation d'une lignée cellulaire, pour la fabrication d'un médicament destiné au traitement du psoriasis.

10. Utilisation conforme à la revendication 9, ladite lignée cellulaire étant une lignée de cellules U937, une lignée de cellules HL60 ou une lignée de cellules M1.

## Revendications (Revendications pour l'(les) Etat(s) contractant(s) suivant(s): ES)

1. Procédé de fabrication d'un médicament destiné au traitement du psoriasis, qui comporte le fait de mélanger avec un véhicule approprié un composé de formule I : 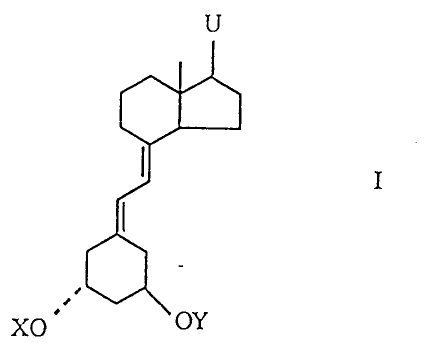 dans laquelle X et Y représentent chacun, indépendamment, un atome d'hydrogène ou un groupe acyle, alkylsilyle ou alcoxyalkyle, et U représente une chaîne latérale de formule 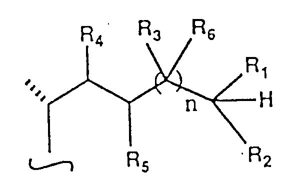 dans laquelle R₁ et R₂ représentent chacun, indépendamment, un groupe alkyle, deutéroalkyle, hydroxyalkyle ou fluoroalkyle, ou bien représentent conjointement un groupe ―(CH₂)ₘ― où m représente un nombre entier valant de 2 à 5, R₃ représente un atome d'hydrogène, de deutérium ou de fluor ou un groupe hydroxyle, O-acyle, alkyle, hydroxyalkyle ou fluoroalkyle, R₆ représente un atome d'hydrogène, de deutérium ou de fluor ou un groupe alkyle, hydroxyalkyle ou fluoroalkyle, ou bien R₃ et R₆ représentent conjointement un atome d'oxygène à double liaison ou un atome de carbone à double liaison, R₄ et R₅ représentent conjointement une triple liaison carbone-carbone et n représente un nombre entier valant de 1 à 5, et l'atome de carbone occupant n'importe laquelle des positions 20, 22 et 23 dans la chaîne latérale peut être remplacé par un atome d'oxygène, de soufre ou d'azote, sous réserve que, si R₃ représente autre chose qu'un groupe hydroxyle ou O-acyle, au moins l'un des R₃ et R₆ représente un atome d'hydrogène ou de deutérium.

2. Procédé conforme à la revendication 1, dans lequel R₃ représente un groupe hydroxyle ou O-acyle.

3. Procédé conforme à la revendication 1 ou 2, dans lequel le médicament contient de 0,01 µg à 100 µg du composé par gramme de médicament.

4. Procédé conforme à l'une des revendications 1 à 3, où le médicament peut être administré à un patient par voie orale ou parentérale, ou encore en usage externe si R₃ représente un groupe hydroxyle.

5. Procédé conforme à l'une des revendications précédentes, où l'on emploie le médicament de façon à apporter ledit composé en une quantité de 0,1 µg/jour à 100 µg/jour.

6. Procédé de fabrication d'un médicament destiné au traitement du psoriasis, qui comporte le fait de mélanger avec un véhicule approprié une 1α-hydroxy-19-nor-vitamine D, composé de formule I définie dans la revendication 1 ou 2, lequel composé est converti, après administration chez l'homme, en un métabolite qui provoque in vitro la différenciation d'une lignée cellulaire.

## Revendications (Revendications pour l'(les) Etat(s) contractant(s) suivant(s): GR)

1. Utilisation d'un composé de formule I pour la fabrication d'un médicament destiné au traitement du psoriasis, la formule I étant la suivante : 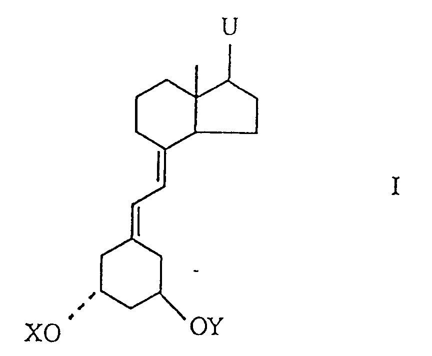 dans laquelle X et Y représentent chacun, indépendamment, un atome d'hydrogène ou un groupe acyle, alkylsilyle ou alcoxyalkyle, et U représente une chaîne latérale de formule 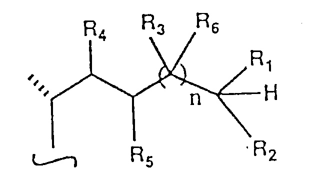 dans laquelle R₁ et R₂ représentent chacun, indépendamment, un groupe alkyle, deutéroalkyle, hydroxyalkyle ou fluoroalkyle, ou bien représentent conjointement un groupe ―(CH₂)ₘ― où m représente un nombre entier valant de 2 à 5, R₃ représente un atome d'hydrogène, de deutérium ou de fluor ou un groupe hydroxyle, O-acyle, alkyle, hydroxyalkyle ou fluoroalkyle, R₆ représente un atome d'hydrogène, de deutérium ou de fluor ou un groupe alkyle, hydroxyalkyle ou fluoroalkyle, ou bien R₃ et R₆ représentent conjointement un atome d'oxygène à double liaison ou un atome de carbone à double liaison, R₄ et R₅ représentent conjointement une triple liaison carbone-carbone et n représente un nombre entier valant de 1 à 5, et l'atome de carbone occupant n'importe laquelle des positions 20, 22 et 23 dans la chaîne latérale peut être remplacé par un atome d'oxygène, de soufre ou d'azote, sous réserve que, si R₃ représente autre chose qu'un groupe hydroxyle ou O-acyle, au moins l'un des R₃ et R₆ représente un atome d'hydrogène ou de deutérium.

2. Utilisation conforme à la revendication 1, pour laquelle R₃ représente un groupe hydroxyle ou O-acyle.

3. Utilisation conforme à la revendication 1 ou 2, où le médicament contient de 0,01 µg à 100 µg du composé par gramme de médicament.

4. Utilisation conforme à l'une des revendications 1 à 3, où le médicament peut être administré à un patient par voie orale ou parentérale, ou encore en usage externe si R₃ représente un groupe hydroxyle.

5. Utilisation conforme à l'une des revendications précédentes, où l'on emploie le médicament de façon à apporter ledit composé en une quantité de 0,1 µg/jour à 100 µg/jour.

6. Utilisation d'une 1α-hydroxy-19-nor-vitamine D, composé de formule I définie dans la revendication 1 ou 2, lequel composé est converti, après administration chez l'homme, en un métabolite qui provoque in vitro la différenciation d'une lignée cellulaire, pour la fabrication d'un médicament destiné au traitement du psoriasis.

7. Utilisation conforme à la revendication 6, ladite lignée cellulaire étant une lignée de cellules U937, une lignée de cellules HL60 ou une lignée de cellules M1.

8. Procédé de préparation d'une composition appropriée pour le traitement du psoriasis par voie orale ou parentérale, qui contient un composé de formule I, définie dans la revendication 1 ou 2, et un véhicule convenable, lequel procédé comporte le fait de mélanger le composé de formule I avec le véhicule.

9. Procédé de préparation d'une composition appropriée, en usage externe, pour le traitement du psoriasis, qui contient un composé de formule I, définie dans la revendication 1, où R₃ représente un groupe hydroxyle, et un véhicule convenable, lequel procédé comporte le fait de mélanger le composé de formule I avec le véhicule.

10. Procédé conforme à la revendication 8 ou 9, dans lequel la composition contient de 0,01 µg à 100 µg dudit composé par gramme de composition.

11. Procédé de fabrication d'un médicament destiné au traitement du psoriasis, qui comporte le fait de mélanger un composé de formule I, définie dans la revendication 1, avec un véhicule approprié.
